# EUROPEAN PATENT APPLICATION

(11) **EP 4 786 571 A1**
(43) Date of publication of application: **05.08.2026**
(21) Application number: 25224035.3
(22) Date of filing: 16.12.2025
(51) Int. Cl.: C12M 1/00, C12M 1/42, C12M 1/34, C12M 1/36

(54) **HIGH THROUGHPUT ENTITIES SCREENING SYSTEM**

(30) Priority: 31.01.2025 EP 25155220
(71) Applicant: Imec VZW, 3001 Leuven (BE)
(72) Inventor: DUCKERT, Bastien, 3001 Heverlee (BE); LAGAE, Liesbet, 3000 Leuven (BE); FAUVART, Maarten, 3060 Bertem (BE); HOELZ, Kathrin, 3001 Leuven (BE); STIRPARO, Rocco, 3000 Leuven (BE)
(74) Representative: Winger

(57) **Abstract**

A method for screening entities with different characteristics for their effect on biological cells of a biological cellular material, comprising: laterally constraining said entities having different characteristics in different locations of said biological cellular material, thereby preventing mixing of said entities having different characteristics; applying a transfection activation field to said different locations to allow said entities to be delivered into said biological cells, thereby allowing said entities to have an effect on said biological cells; and detecting said effect.

## Description

### Field of the Invention

The present invention relates to the field of high-throughput screening of biologically active entities, and in more specific embodiments, to a method and system for screening nucleic acids using gene electrotransfer on a microelectrode array.

### Background of the Invention

Nucleic acid delivery into cells is a fundamental technique in molecular biology and biotechnology. It enables the introduction of foreign genetic material into cells for various applications, including gene therapy, functional genomics, and drug discovery. Traditional methods for nucleic acid delivery have relied on chemical transfection reagents or bulk electroporation techniques.

Electroporation is typically performed in bulk, where high voltage pulses are applied to cell suspensions in a cuvette to temporarily permeabilize cell membranes, allowing nucleic acids to enter. The cuvette contains two electrode plates across which a substantial electrical potential is applied.

In traditional cuvette electroporation, each nucleic acid solution and cell suspension mixture must be processed in separate cuvettes, each equipped with its own pair of electrode plates. This requirement limits the potential for miniaturization and scaling up the screening of molecules. Additionally, cuvette electroporation demands a large number of cells, significant volumes of nucleic acid solution, and considerable manual effort. Furthermore, this method often results in significant cell death due to the high voltages used and necessitates large quantities of cells and nucleic acids.

These requirements hinder its suitability for screening large numbers of distinct nucleic acids efficiently.

Chemical transfection, also called reversed transfection, involves complexing nucleic acids with a chemical transfection reagent (e.g., cationic lipids) to facilitate cellular uptake. When cells are dispensed in bulk grown on the surface on which the complexes are present, the solubilization of the transfection mix in the cell culture medium triggers the transfection of the cells. While this widely used concept can allow for higher throughput screenings than bulk cuvette electroporation, chemical transfection can suffer from low transfection efficiency, high cytotoxicity, and limited applicability across different cell types.

There is an ongoing need for improved methods to deliver nucleic acids into cells with high efficiency and low toxicity. In particular, the ability to screen large libraries of nucleic acid constructs in a high-throughput manner remains challenging with existing techniques. Current approaches like reverse transfection arrays allow parallel testing of multiple constructs, but are limited by the low efficiency of chemical transfection methods.

Improved methods for high-throughput, efficient, and cell-type independent nucleic acid delivery would accelerate many areas of biomedical research and development. Applications could include rapid screening of gene therapies, optimization of CRISPR gene editing components, and discovery of novel RNA therapeutics. Despite progress in the field, there remains a need for further technological advancements in methods for screening entities (e.g., nucleic acids) with different characteristics for the effect of these entities on biological cells that overcome at least partially one or more of the above limitations.

### Summary of the Invention

It is an object of embodiments of the present invention to provide a high-throughput method for screening nucleic acids delivered into cells. This objective is accomplished by a method for screening entities with different characteristics for their effect on biological cells according to the invention.

In the first aspect, the present invention relates to a method for screening entities with different characteristics for the effect of these entities on biological cells of a biological cellular material, comprising:
a. Laterally constraining said entities having different characteristics in different locations of said biological cellular material, thereby preventing mixing of said entities having different characteristics,
b. Applying a transfection activation field to said different locations in such a way as to allow said entities to be delivered into said biological cells of said biological cellular material, thereby allowing said entities to have an effect on said biological cells, and
c. Detecting said effect.

In embodiments, the entities may be molecules or compounds, preferably nucleic acids such as DNA or RNA (e.g., guide RNAs for CRISPR-Cas9 or plasmids). More preferably the nucleic acids may be oligonucleotides such as guide RNAs for CRISPR-Cas9. This allows for screening of a wide range of biologically relevant molecules.

In embodiments, the entities may be provided by physically dispensing them onto the substrate, or they may be synthesized on the spot (in situ) directly at predetermined locations on the substrate.

In embodiments, the biological cellular material may be on a substrate and wherein step a of laterally constraining said entities comprises attaching them in different locations of a surface of said substrate, said surface physically contacting said biological cellular material. This provides a convenient platform for constraining the entities. Also, this does not require compartmentalization, which itself requires much space.

In embodiments, step a may comprise:
a1. Laterally constraining said entities (10) on a substrate (100) at said different locations (40), and subsequently
a2. Providing said biological cellular material over said substrate (100).

In embodiments, step a2 may be followed by allowing the biological cells (20) of said biological cellular material to adhere to said substrate (100). The biological cells (20) are allowed to adhere to the substrate (100) at said different locations (40) where said entities (10) are constrained.

In embodiments, said substrate (100) may comprise a multielectrode array (130) with electrodes (140) at said different locations (40), and said biological cells (20) are allowed to adhere to said electrodes (140).

In embodiments, the biological cellular material may be distributed in compartments (e.g., wells) present on a substrate, and wherein step a of laterally constraining said entities comprises distributing them in said compartments. This allows for physical separation of different entity-cell combinations. Also, this does not require chemical attachment to the substrate, which itself adds complexity to the method.

In embodiments, the compartments present on the substrate may be made of non-conductive CMOS-compatible materials. This is advantageous as it allows the formation of a very large density of compartments by using CMOS technology. For instance, more than 1000 compartments can be formed on or in the substrate.

In embodiments, the density of compartment formed on or in the substrate may be at least 100 per cm².

In embodiments, the non-conductive CMOS-compatible materials can be selected from silicon dioxide (SiO₂), silicon nitride (Si₃N₄), silicon oxynitride (SiON), silicon carbide (SiC), hafnium oxide (HfO₂), aluminum oxide (Al₂O₃), SiCOH, photoresists, polyimides, SU-8, siloxane polymers, borosilicate glass, phosphosilicate glass (PSG), boron-phosphosilicate glass (BPSG), Si, and Ge, amongst others.

In embodiments, the substrate may comprise a multielectrode array and wherein step b and/or step c are performed by means of electrodes of said multielectrode array. This enables precise control over transfection and detection.

In embodiments, the multielectrode array may comprise individually addressable electrodes.

In embodiments, step b may be performed by means of said electrodes, wherein said entities are charged entities, wherein the microelectrode array comprises individually addressable electrodes made of an electrically-conductive CMOS-compatible material having an oxide top layer, e.g., a native oxide top layer, and wherein step a comprises electrostatically coupling said charged entities to the oxide top layer. This allows for controlled immobilization and release of entities.

In embodiments, step b may be performed simultaneously to said different locations.

In embodiments, the charged entities may be negatively charged entities electrostatically attached to positively charged carriers, e.g., molecules, themselves directly attached to the oxide top layer. This provides a stable, yet reversible, immobilization strategy.

In embodiments, the electrically-conductive CMOS-compatible material may be a metal, a metal nitride, or a metal oxide, preferably a metal or a metal nitride. Examples of metal oxides are iridium oxide and ruthenium oxide. This enables integration with standard semiconductor fabrication.

In embodiments, the electrically-conductive CMOS-compatible material may be selected from the group consisting of Titanium Nitride, Ruthenium, Platinum, Iridium, Ruthenium oxide and Iridium Oxide. These materials provide good electrical and biocompatibility properties. Preferably, the CMOS-compatible material is selected from the group consisting of Titanium Nitride, Ruthenium, Platinum, and Iridium.

In embodiments, the positively charged molecules may be Poly(ethylene-imine) (PEI). PEI is an effective transfection agent.

In embodiments, the biological cellular material may be a cell culture or a tissue. This allows screening in different biological contexts.

In embodiments, the effect on the biological cells may be detected by imaging, e.g., fluorescence imaging and/or lens-free imaging. These are sensitive detection methods.

In embodiments, the method may further comprise the step of creating a dataset comprising said characteristics of said entities associated with the effects detected for said entities. This enables systematic analysis of results.

In embodiments, the method may further comprise a step of analyzing the dataset, e.g., using a machine learning algorithm, to correlate said effects with characteristics of the entities associated with said effect. This allows for identification of trends and patterns.

In embodiments, the method may further comprise a step of designing new entities based on the analysis of the dataset. This enables iterative optimization.

In embodiments, the method may further comprise a step of repeating steps a to c while using the new entities in step a. This allows for multiple rounds of screening and optimization.

In embodiments, the laterally constraining step a may comprise using an arraying tool, e.g., a spotting tool, to place the entities in the different locations. This enables precise spatial control over entity placement.

In embodiments, the transfection activation field may be individually applied to each of said different locations in a controlled manner, taking into account one or more cellular health factors so as to enable delivery of said entities into said biological cells while preventing cellular death directly caused by the transfection activation field. This optimizes transfection efficiency while maintaining cell viability.

In embodiments, the method may further comprise measuring the permeability of the cell membrane at said different locations, e.g., above each electrode when electrodes are present, and wherein the applied transfection activation field is adjusted based on the measured permeability of the cell membrane. This allows for real-time optimization of transfection parameters.

In embodiments, the effect on the biological cells may be a change in cell morphology, viability, or gene expression. These are relevant biological readouts.

In embodiments, step a may comprise first a1. laterally constraining said entities having different characteristics on different electrodes of a multielectrode array, then a2. selectively removing some of said entities by applying an electrical field to some of the electrodes, then a3. Providing the biological cellular material over the multielectrode array. This allows for precise patterning of entities.

In embodiments, before step a, the substrate, e.g., the multielectrode array, may be cleaned by UV-ozone and/or by a wet cleaning. This ensures a clean surface for entity immobilization.

In embodiments, said transfection activation field may be selected from the group consisting of electrical field, photoporation field, and acoustic field. These are effective methods for cellular transfection.

In embodiments, the transfection activation field may be an electrical field, and the delivery of said entities into said biological cells is by electroporation. Electroporation is an efficient transfection method.

In embodiments, said transfection activation field may not be a chemical transfection method. This avoids potential toxicity issues associated with chemical transfection reagents.

Any feature of the first aspect may be as correspondingly described in any embodiment of the second aspect.

In the second aspect, the present invention relates to a system for screening entities with different characteristics for their effect on biological cells of a biological cellular material, comprising:
- a device comprising:
   ∘ A substrate for supporting the biological cellular material,
   ∘ Constraining means for laterally constraining said entities over different locations of said substrate so as to prevent the mixing of entities differing in their characteristics,
   ∘ Means for applying a transfection activation field to the different locations in such a way as to allow said entities to be delivered into said biological cells of said biological cellular material, thereby allowing said entities to have an effect on these biological cells,
   ∘ A detector for detecting said effect, and
- A control unit configured to pilot the device so that it performs steps b and c of the method according to any embodiment of the first aspect.

In embodiments, the system may further comprise a delivery unit, and wherein the control unit is further configured to pilot the delivery unit so that it delivers said entities over said substrate so that entities differing in their characteristics are constrained over different locations of the substrate by the constraining means. This enables automated entity placement.

In embodiments, the delivery unit may comprise an arraying tool, e.g., a spotting tool, and wherein the control unit is further configured to pilot the arraying tool to place the entities in the different locations. This allows for precise spatial control over entity placement.

In embodiments, said substrate may comprise a multielectrode array, wherein said means for applying a transfection activation field are means for applying an electrical field, and wherein said means for applying an electrical field and/or said detector comprises electrodes of the multielectrode array. This enables integrated transfection and detection.

In embodiments, the means for applying the transfection activation field may be configured to individually apply the transfection activation field to each of said different locations in a controlled manner, taking into account one or more cellular health factors so as to enable delivery of the entities into said biological cells while preventing cellular death directly caused by the transfection activation field. This optimizes transfection efficiency while maintaining cell viability.

In embodiments, the constraining means may comprise compartments on the substrate. This allows for physical separation of different entity-cell combinations.

In embodiments, the detector may comprise an imaging unit, such as a fluorescence imaging unit and/or a lens-free imaging unit. These are sensitive detection devices.

In embodiments, the control unit may be further configured for collecting and processing data from the detector to create a dataset that maps the observed effects to the corresponding locations on the substrate. This enables systematic analysis of results.

In embodiments, the control unit may be further configured for correlating the effects observed with the specific entities constrained at those locations, thereby providing correlation between the characteristics of the entities and their effects on the biological cells. This allows for identification of trends and patterns.

In embodiments, the system may further comprise means for designing new entities based on the correlation between the characteristics of the entities and their effects on the biological cells. This enables iterative optimization.

In embodiments, the control unit may be further configured to repeat steps a through c while using said new entities in step a. This allows for multiple rounds of screening and optimization.

Any feature of the second aspect may be as correspondingly described in any embodiment of the first aspects.

It is an advantage of embodiments of the present invention that a high-throughput screening of entities delivered into cells can be achieved. It is a further advantage of embodiments of the present invention that different entities can be pre-arrayed on a high-density microelectrode array designed for electroporation of cells grown atop its electrodes. It is an advantage of embodiments of the present invention that entities can be released from the chip and delivered into cells upon submission of a transfection activation field. It is a further advantage of embodiments of the present invention that a largely automated and plug-and-play operation can be implemented. It is an advantage of embodiments of the present invention that tens of thousands of different entities can be screened within a single week. It is a further advantage of embodiments of the present invention that high delivery efficiencies and low cytotoxicity can be achieved compared to chemical transfection or bulk electroporation. It is an advantage of embodiments of the present invention that feedback control of the cell delivery process can be implemented by measuring cell membrane permeability. It is a further advantage of embodiments of the present invention that CMOS-compatible electrode materials can be used. It is an advantage of embodiments of the present invention that spatial selectivity in nucleic acid delivery can be achieved through the use of individually addressable microelectrodes. It is a further advantage of embodiments of the present invention that the workflow can be simplified for the user with minimal handling steps required. It is a further advantage of embodiments of the present invention that combinations of different entities, such as different guide RNAs, can be co-delivered into a single cell by constraining them at neighboring locations, enabling deterministic screening of combinatorial effects that are difficult to assess with conventional pooled screening methods.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a flowchart of a method according to embodiments of the present invention.
Fig. 2 is a flowchart of a method according to embodiments of the present invention.
Fig. 3 is a flowchart of a method according to embodiments of the present invention.
Fig. 4 is a block diagram of a system according to embodiments of the present invention.
Fig. 5 is a schematic diagram illustrating a transfection screening workflow according to embodiments of the present invention.
Fig. 6 is a schematic diagram illustrating plasmid DNA immobilization on an electrode according to embodiments of the present invention.
Fig. 7 is a schematic diagram illustrating steps of a screening method according to embodiments of the present invention.

In the different figures, the same reference signs refer to the same or analogous elements.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

As used herein, and unless otherwise specified, the term *"entities"* refers to any discrete units or substances that can be applied to or interact with biological cells, including but not limited to molecules, compounds, particles, or organisms. Examples of entities include nucleic acids, proteins, small molecules, nanoparticles, viruses, or bacteria.

As used herein, and unless otherwise specified, the term *"entities with different characteristics"* refers to distinct entities that vary in one or more properties relevant to their interaction with or effect on biological cells. Examples of different characteristics include, but are not limited to, variations in chemical structure, sequence, size, charge, binding affinity, biological activity, or intended cellular target. In the context of nucleic acids, different characteristics may include variations in sequence, length, modifications, or encoded products. For proteins, different characteristics may include variations in amino acid sequence, post-translational modifications, or structural conformations. The term encompasses any set of entities where each member possesses at least one feature that distinguishes it from the others in the set, in a way that may produce different effects when interacting with biological cells.

As used herein, and unless otherwise specified, the term *"for the effect of these entities on biological cells"* refers to any measurable or observable change in the state, behavior, or properties of biological cells resulting from their interaction with the entities being screened. These effects may be immediate or delayed. These effects may be transient or permanent. These effects may occur at the molecular, cellular, or phenotypic level. Examples of such effects include, but are not limited to, changes in gene expression, protein production, metabolic activity, cell morphology, growth rate, viability, differentiation state, signaling pathway activation, or any other cellular process. The term also encompasses more complex cellular responses such as the activation of specific genetic programs, alterations in cell fate decisions, or modulation of cellular functions. In the context of this invention, these effects serve as the basis for evaluating and comparing the biological activities or potencies of the different entities being screened.

As used herein, and unless otherwise specified, the term *"biological cellular material"* refers to any collection of biological cells, whether in a monolayer or three-dimensional structure. Examples of biological cellular material include cell cultures, tissues, and organoids.

As used herein, and unless otherwise specified, the term *"laterally constraining"* refers to the act of physically restricting or confining entities to specific locations or regions in a horizontal plane, preventing their movement or mixing with entities in other locations. Examples of laterally constraining include attaching entities to specific surface areas (e.g., of a substrate) on which the biological cellular material is present or will be provided, confining entities within compartments or wells in which the biological cellular material is present or will be provided, or using physical barriers to separate different entity populations.

As used herein, and unless otherwise specified, the term *"transfection activation field"* refers to any physical stimulus or energy field applied to facilitate the formation of pores in a biological cell membrane for allowing the entry of entities into the biological cell. This field can also be called a "pore creation field". Examples of transfection activation fields include electrical fields for electroporation, light for photoporation, or acoustic waves for sonoporation.

As used herein, and unless otherwise specified, the phrase *"in such a way as to allow said entities to be delivered into said biological cells"* refers to the application of a transfection activation field that facilitates the entry of the entities into the interior of the biological cells. This process may involve temporarily altering the permeability of the cell membrane to allow the passage of the entities from the extracellular environment into the cytoplasm or other intracellular compartments. The delivery method may be designed to be efficient while minimizing damage to the cells. In the context of this invention, this phrase encompasses various techniques for introducing entities into cells. A preferred such technique is electroporation using microelectrode arrays. The delivery process may be controlled and optimized to ensure that the entities can cross the cell membrane barrier and reach the intracellular space where they can exert their biological effects. This may involve one or more of the following: the control of the strength of the applied field, the control of the duration of the applied field, and the control of the timing of entity release from the substrate.

As used herein, and unless otherwise specified, the phrase *"detecting said effect"* refers to the process of observing, measuring, or quantifying the changes induced in the biological cells by the delivered entities, specifically using methods that are compatible with cells remaining on the substrate. This detection is preferably primarily performed through non-invasive or minimally invasive techniques that do not require removal or destruction of the cells from the microelectrode array. Examples include, but are not limited to, live-cell fluorescence microscopy, e.g., to visualize protein expression or cellular localization, label-free imaging techniques such as phase contrast or holographic microscopy, impedance measurements using a microelectrode array to assess cellular properties, bioluminescence imaging for reporter gene expression, and fluorescence-based assays for metabolic activity or membrane integrity that can be performed in situ. The detection may also involve the use of cell-permeable dyes or sensors that can be added to the culture medium. Techniques that require removal of cells from the substrate, such as flow cytometry, or that involve cell lysis, such as Western blotting or RT-qPCR, are preferably excluded from this definition in the context of this invention. The detection process is preferably designed to be high-throughput, allowing for the simultaneous assessment of effects across multiple locations on the substrate, and may involve real-time monitoring or defined time-point measurements to capture the range of cellular responses to the delivered entities.

As used herein, and unless otherwise specified, the term *"multielectrode array"* refers to a device comprising multiple individually addressable electrodes arranged in a pattern on a substrate. Examples of multielectrode arrays include planar electrode arrays, 3D electrode arrays, or microelectrode arrays integrated with CMOS circuitry.

As used herein, and unless otherwise specified, the term *"CMOS-compatible material"* refers to materials that can be used in the fabrication of complementary metal-oxide-semiconductor (CMOS) devices without contaminating or interfering with the CMOS manufacturing process.

As used herein, and unless otherwise specified, the term *"dataset"* refers to a structured collection of data that associates the characteristics of entities with their observed effects on biological cells. Examples of datasets include databases, spreadsheets, or data files containing information on entity properties, cellular responses, and experimental conditions.

As used herein, and unless otherwise specified, the phrase *"designing new entities (380) based on the analysis of the dataset"* refers to the process of using the information gathered from previous screening rounds to inform the creation of new entities for subsequent testing. These new entities may be created virtually. These entities may also be synthesized. This process may involve interpreting the correlations between entity characteristics and their observed effects on biological cells, as captured in the dataset, to generate hypotheses about which features contribute to desired outcomes. The design process may employ various computational methods, including but not limited to, machine learning algorithms, statistical modeling, structure-activity relationship analyses, or rational design approaches. For nucleic acids, this might involve modifying sequences, altering structural elements, or introducing chemical modifications. For proteins, it could include amino acid substitutions, domain swapping, or changes in post-translational modifications. The goal is to iteratively refine and optimize the entities based on empirical data, potentially leading to entities with enhanced efficacy, reduced off-target effects, or novel functionalities. This design process is integral to the iterative nature of embodiments of the screening method, allowing for the continuous improvement and evolution of the entities being tested.

As used herein, and unless otherwise specified, the term *"control unit"* refers to a hardware or an integrated hardware and software system configured to pilot the device so that it performs steps b and c of the method according to any embodiment of the first aspect. It may be designed to manage, coordinate, and automate the various components and processes of the screening system. The control unit may be configured for orchestrating a sequence of operations comprising one or more of the following: controlling the application of the transfection activation field to specific locations on the substrate, managing the timing and parameters of entity delivery, coordinating detection systems, collecting and processing data, and adjusting experimental parameters in real-time based on feedback. It may incorporate microprocessors, field-programmable gate arrays (FPGAs), or other computational hardware, along with specialized software algorithms for data analysis, machine learning, and process optimization. The control unit may interface with the means for applying a transfection activation field, detection systems, and any auxiliary equipment, enabling precise temporal and spatial control over the screening process. It may also manage data storage, organization, and retrieval, facilitating the creation and analysis of the dataset that maps entity characteristics to observed effects. In advanced implementations, the control unit may incorporate adaptive learning algorithms to optimize screening parameters based on accumulated data, enhancing the efficiency and effectiveness of the screening process over time. The control unit is advantageous to achieve high-throughput, automatization of the screening system, minimizing the need for manual intervention, and ensuring reproducibility across experiments.

As used herein, and unless otherwise specified, the term *"UV-ozone and*/*or wet cleaning"* refers to surface preparation techniques used to clean and activate the substrate, particularly the microelectrode array, prior to the immobilization of entities. UV-ozone cleaning involves exposing the substrate surface to ultraviolet light in an oxygen-rich environment, generating highly reactive ozone molecules that oxidize and remove organic contaminants from the surface. This process also activates the surface by creating oxygen-containing functional groups, which can enhance subsequent binding of entities or other molecules. Wet cleaning, in the present context, refers to the use of liquid chemical solutions to remove contaminants and prepare the surface. This may include treatments with acids, bases, oxidizers, or organic solvents, followed by thorough rinsing, typically with pure water. The wet cleaning agents is preferably selected to be compatible with the electrode materials and to avoid damage to any underlying electronics. These cleaning methods may be used individually or in combination, depending on the specific requirements of the substrate and the entities to be immobilized. The cleaning process is advantageous for ensuring consistent and efficient immobilization of entities across the array, as well as for maintaining the performance and reliability of the microelectrodes. Unlike more aggressive cleaning methods such as oxygen plasma, which may damage sensitive CMOS-compatible electrode materials or underlying electronics, UV-ozone and appropriate wet cleaning protocols are designed to effectively prepare the surface while preserving the integrity of the microelectrode array.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

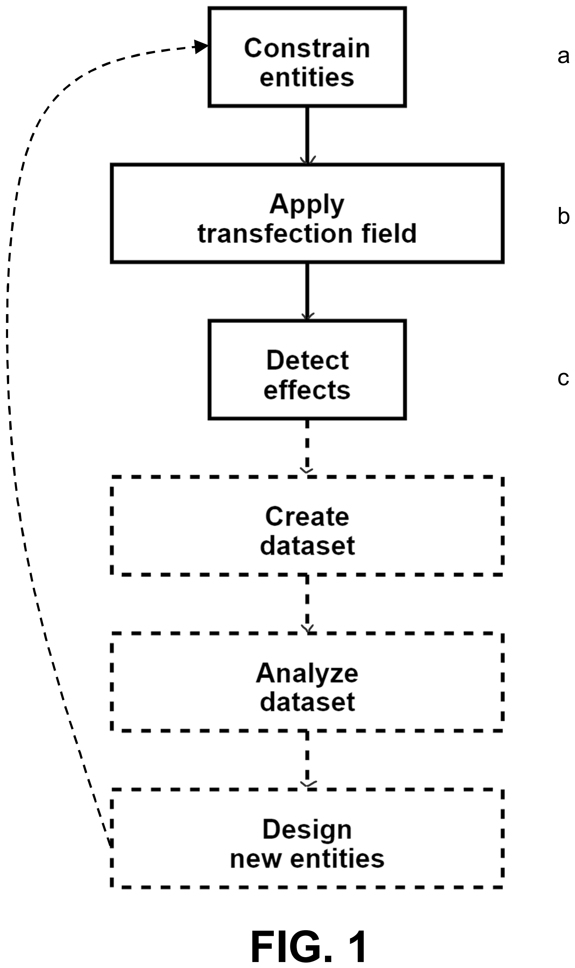

We now refer to Fig. 1, which illustrates a flowchart of a method for screening entities (10) with different characteristics for the effect of these entities (10) on biological cells (20) of a biological cellular material according to embodiments of the present invention. The method comprises the steps of: laterally constraining the entities (10) having different characteristics in different locations (40) of the biological cellular material, thereby preventing mixing of the entities (10) having different characteristics; applying a transfection activation field to the different locations (40) in such a way as to allow the entities (10) to be delivered into the biological cells (20) of the biological cellular material, thereby allowing the entities (10) to have an effect on the biological cells (20); and detecting the effect. The method further comprises the steps of: creating a dataset (360) comprising the characteristics of the entities (10) associated with the effects detected for the entities (10); analyzing the dataset (360), e.g., using a machine learning algorithm (370), to correlate the effects with characteristics of the entities (10) associated with the effect; designing new entities (380) based on the analysis of the dataset (360); and repeating the steps of laterally constraining the entities (10), applying the transfection activation field, and detecting the effect while using the new entities (380) in the laterally constraining step.

In embodiments, rather than dispensing already-synthesized entities onto the substrate, the entities (e.g., nucleic acids, peptides, or small molecules) may be synthesized in situ at each of the different locations. For example, microarray-based DNA synthesis techniques can generate a variety of oligonucleotides directly on the substrate at predetermined spots. Alternatively, peptides or small molecules could be assembled on-site using patterned microfluidics or localized reagent delivery, ensuring that the resulting entities remain strictly confined to their respective locations. This embodiment obviates the need for external synthesis and transport of each individual entity, thereby greatly streamlining the workflow for large-scale library screening.

In embodiments, the system may be configured to achieve single-cell resolution by designing the different locations (40), e.g., the electrodes of a multielectrode array (130), to be of a size comparable to or smaller than a single biological cell (20). In this configuration, the biological cellular material is provided at a density such that individual cells (20) settle onto one or more individual locations (40). A detection step, for instance using the imaging unit (600), can be used to identify the locations occupied by a single cell. The control unit (610) is then configured to apply the transfection activation field selectively only to these identified locations. This allows for the delivery of a single type of entity into a single cell. This embodiment is particularly advantageous for applications requiring precise cellular manipulation and analysis, such as in cell line development, high-purity functional genomics screening, or studying cellular heterogeneity in response to a specific therapeutic agent.

In embodiments, step a may be performed so that at least one cell transfected in step b is transfected with only a single type of entity.

In embodiments, step a may be performed so that a majority of the cells transfected in step b are transfected with only a single type of entity. This may be accomplished, for example, by dispensing the entities at a suitable concentration. This may be accomplished, for example, by dispensing the entities at concentration, such that the probability of more than one entity occupying a location suitable to transfect a same biological cell (20) is minimized according to a Poisson distribution. For instance, a sub-femtomolar concentration may be used. The delivery of a single active molecule enables the ultimate level of precision in biological screening. This is advantageous for investigating stochastic effects in gene expression, understanding the minimal threshold for a biological response, or assessing the potency of single-molecule therapeutics.

In embodiments, step a may be performed so that at least one cell transfected in step b is transfected with two or more different entities (10) with different characteristics.

In embodiments, step a may be performed so that a majority of the cells transfected in step b are transfected with a combination of two or more different entities (10) with different characteristics. This may be accomplished, for example, by dispensing the entities at a suitable concentration.

In embodiments, the different locations for constraining entities may be arranged with a density high enough such that a single biological cell (20) is positioned to be in simultaneous contact with two or more of said different locations. For instance, when using a microelectrode array (130), the electrode pitch may be smaller than the diameter of a biological cell. This configuration enables a powerful application of the invention: the co-delivery of two or more different entities into the same cell, either simultaneously or in a controlled temporal sequence. By applying the transfection activation field to the two or more locations (e.g., via electrodes) beneath a single cell, a predefined combination of entities (e.g., two different CRISPR guide RNAs) can be co-delivered.

This capability addresses a significant challenge in functional genomics, particularly in combinatorial screening. For example, testing combinations of gene knockouts to overcome drug resistance in cancer is particularly advantageous, as cancers often develop resistance to single-drug therapies. Conventional methods like pooled CRISPR assays are impractical for identifying the effects of combinations of guide RNAs, as this would require screening billions of cells to find statistically significant depletions. The present invention overcomes this limitation by enabling deterministic, arrayed screening where selected combinations of entities are constrained on neighboring locations suitable to transfect a same biological cell. This allows for the direct and efficient testing of synergistic or antagonistic interactions.

In embodiments, when step a is performed so that a majority of the cells transfected in step b are transfected with a combination of two or more different entities (10) with different characteristics (e.g., when the different locations for constraining entities are arranged with a density high enough such that a single biological cell (20) is positioned to be in simultaneous contact with two or more of said different locations), the method may further comprise a step of creating a dataset (360) comprising said the characteristics of said combinations of entities (10) associated with the effects detected for said combinations of entities (10), a step of analyzing the dataset, e.g., using a machine learning algorithm, to correlate said effects with characteristics of the combinations of entities associated with said effect. Here, said effects are the result of the presence of more than one type of entity (i.e., different entities having different characteristics) in a single cell. This allows for identification of trends and patterns (e.g., in the form of a predictive model). The result of the analysis of the dataset may be a predictive model.

In embodiments, the method may further comprise a step of proposing new combination of entities (10) based on the analysis of the dataset (e.g., based on the predictive model). This enables iterative optimization. In other words, the correlation of said effects with the characteristics of the combination of entities associated with said effect (e.g., in the form of a predictive model), may then be used to propose new combinations of entities to be transfected into a single biological cell (20) positioned to be in simultaneous contact with two or more of said different locations where said different entities reside. As it is often impossible to test all possible combinations, the structured data generated from the arrayed combinatorial screen can be used to train predictive models. These models can then propose new, more effective combinations to test in subsequent, rapid screening cycles, creating a powerful closed-loop system for accelerating the discovery of complex biological interactions and refining therapeutic strategies.

In embodiments, the method may further comprise a step of repeating steps a to c while using the new combination of entities in step a. This allows for multiple rounds of screening and optimization.

This deterministic, arrayed format is exceptionally well-suited for integration with machine learning (ML) or artificial intelligence (AI) workflows. As it is often impossible to test all possible combinations.

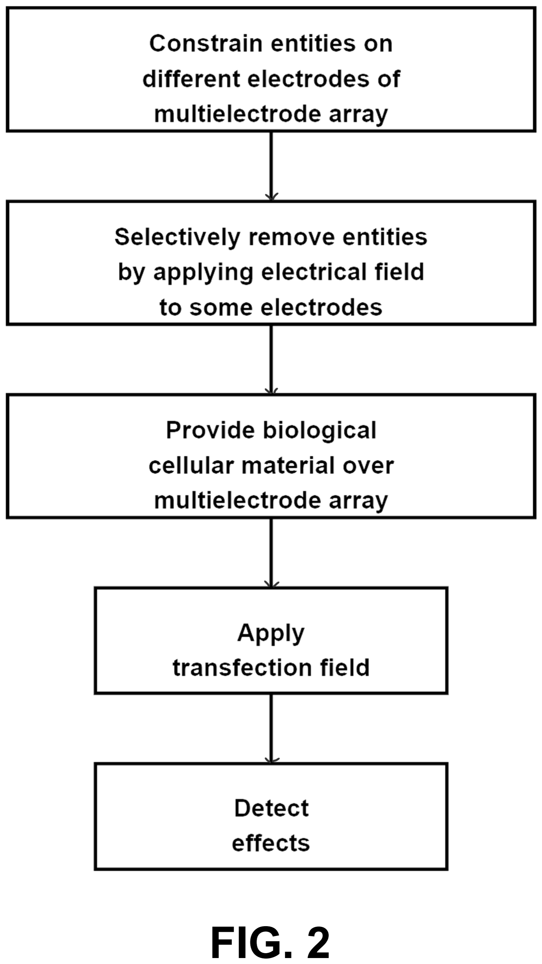

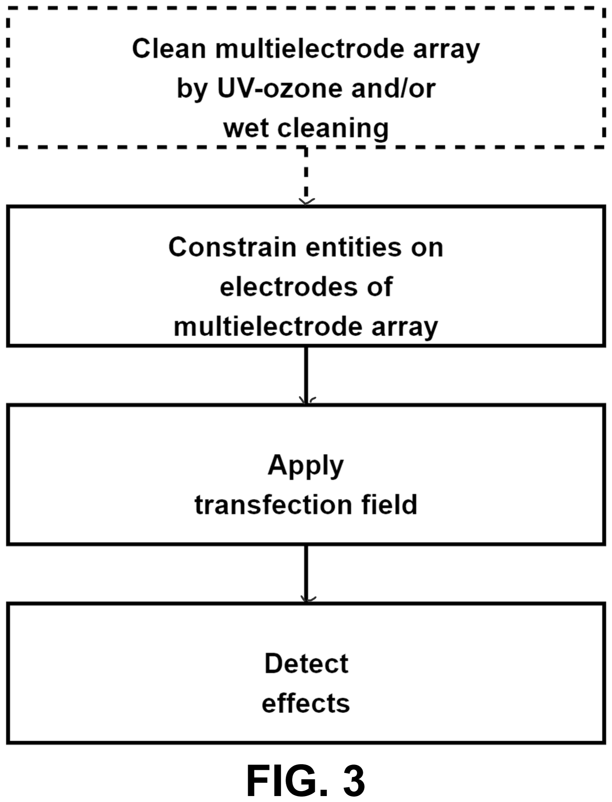

We now refer to Fig. 2, which illustrates a flowchart of a method for screening entities (10) with different characteristics for the effect of these entities (10) on biological cells (20) of a biological cellular material according to embodiments of the present invention where the entities (10) having different characteristics may be laterally constrained on different electrodes (140) of a multielectrode array (130), wherein some of the entities (10) may be selectively removed by applying an electrical field (480) to some of the electrodes (140), and wherein the biological cellular material may then be provided over the multielectrode array (130) before the transfection field is applied and the effects are detected. In Fig.3, the introduction of an optional cleaning step of the substrate, e.g., of the multielectrode array is introduced before step a. This step may be performed by UV-ozone and/or wet cleaning.

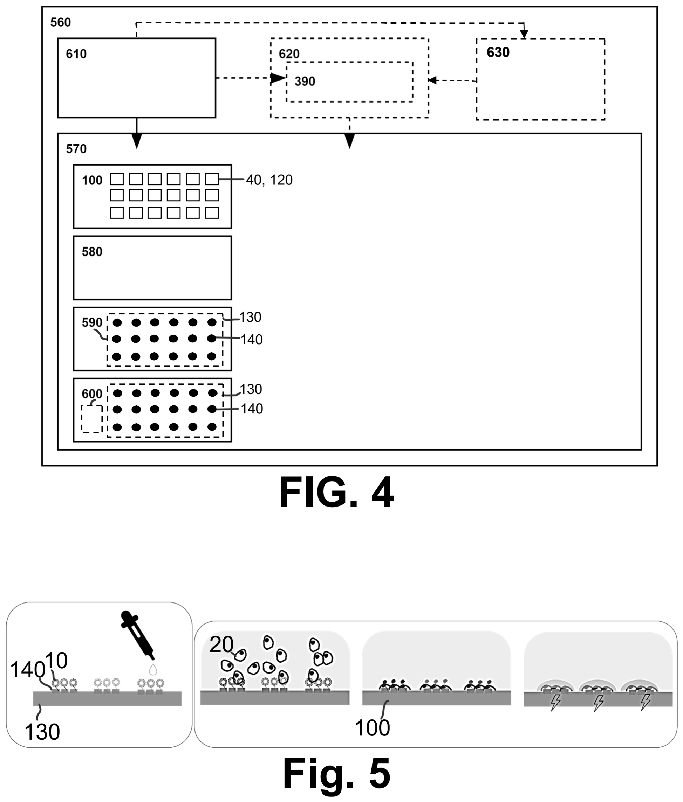

We now refer to Fig. 4, which illustrates a block diagram of a system (560) for screening entities (10) with different characteristics for their effect on biological cells (20) of a biological cellular material according to embodiments of the present invention. The system (560) comprises: a device (570) comprising a substrate (100) for supporting the biological cellular material, constraining means (580) for laterally constraining the entities (10) over different locations (40) of the substrate (100) so as to prevent the mixing of entities (10) differing in their characteristics, means (590) for applying a transfection activation field to the different locations (40) in such a way as to allow the entities (10) to be delivered into the biological cells (20) of the biological cellular material, thereby allowing the entities (10) to have an effect on these biological cells (20), and a detector (600) for detecting the effect; and a control unit (610) configured to pilot the device (570) so that it performs the steps of applying the transfection activation field and detecting the effect. The constraining means (580) may, for instance, comprise compartments (120) on the substrate (100). The means for applying a transfection activation field may, for instance, be a multielectrode array (130) comprising electrodes (140).

The control unit (610) may be configured to apply the transfection activation field to neighboring locations simultaneously, or in a temporal sequence. This allows studying time-dependent interactions.

The system is depicted as optionally comprising a delivery unit (620), wherein the control unit (610) is further configured to pilot the delivery unit (620) so that it delivers said entities (10) over said substrate (100) so that entities (10) differing in their characteristics are constrained over different locations (40) of the substrate (100) by the constraining means (580). The delivery unit is depicted as optionally comprising an arraying tool (390), e.g., a spotting tool, wherein the control unit (610) is further configured to pilot the arraying tool (390) to place the entities in the different locations (40). This allows for precise spatial control over entity placement. The system is further depicted as optionally comprising means (630) for designing new entities based on the correlation between the characteristics of the entities (10) and their effects on the biological cells (20).

We now refer to Fig. 5, which illustrates a schematic of a transfection screening workflow based on DNA immobilization on a microelectrode array (130) according to embodiments of the present invention. Different nucleic acids (10) are individually spotted and immobilized on the electrodes (140) of the microelectrode array (130). After cell seeding in bulk on the chip and cell adhesion to the electrode (140), sending electric pulses will both release the nucleic acids (10) from the electrodes (140) and electroporate the cells (20). During electroporation, cells (20) are permeable to nucleic acids (10) only during the submission of an electric field and will not pick-up free-floating nucleic acids (10) after the electroporation step. Cells (20) will be transfected only with the nucleic acid (10) on top of which they were adhering, thus achieving a spatially resolved transfection with multiple nucleic acids (10), the spatial resolution resulting from local spotting of nucleic acids (10). After transfection, the cells (20) will show a change of behavior corresponding to the nucleic acid (10) above which they grew. For instance, cells (20) may show a different fluorescent color depending on their location, that encodes the identity of the nucleic acid (10) that was delivered. In embodiments, thanks to the selective control of the electrodes on the microelectrode array, the spatial resolution of the DNA immobilization could be improved beyond the resolution of liquid dispensing tools, for instance by using electrical pulses to remove DNA from selected electrodes.

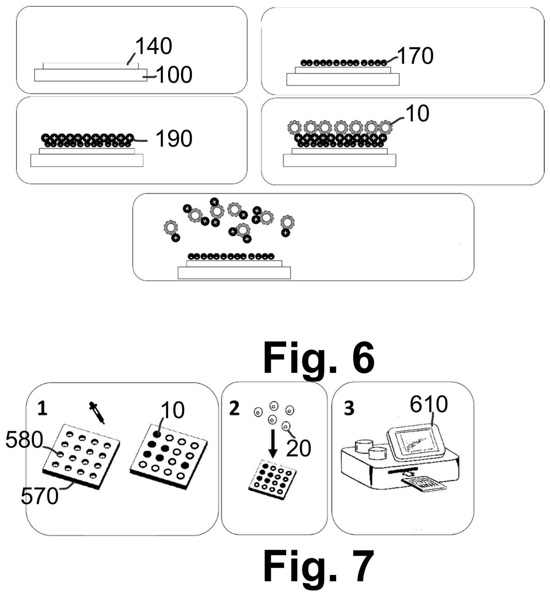

We now refer to Fig. 6, which illustrates a schematic of plasmid DNA immobilization on the native oxide layer (170) of a ruthenium or titanium nitride electrode (140) according to embodiments of the present invention. The plasmid DNA (10), which are negatively charged entities (10), are electrostatically attached to positively charged carriers (190), e.g., Poly(ethylene-imine) (PEI) molecules, themselves directly attached to the oxide top layer (170) of the electrode (140). The electrode (140) is made of an electrically-conductive CMOS-compatible material, such as ruthenium or titanium nitride, having the native oxide top layer (170). Thanks to this non-covalent interaction, the plasmids can be released by applying weak electric pulses to the electrodes (140). In embodiments, the electrode materials may be different. CMOS-compatible electrode materials used may include Titanium (or Titanium Nitride), Platinum, Ruthenium, or Iridium. ITO is not compatible with CMOS, meaning ITO has issues with diffusion to substrate and is not easily combinable with electronic circuits beneath the ITO electrode. In embodiments, the surface preparation may be different: The cleaning strategy used in prior art (oxygen plasma) may be avoided with the devices of the present invention, as it can damage the chip (overoxidation of the electrodes or electrically damaging the CMOS electronics). UV-Ozone and chemical cleaning may be looked at instead (see Fig. 3).

We now refer to Fig. 7, which illustrates a schematic of an example workflow for screening nucleic acids (10) using a pre-dispensed microelectrode array device (570) according to embodiments of the present invention. The pre-dispensing of nucleic acids (10) on chip will be performed in advance, in a fabrication facility for instance, before the device (570) is shipped to the user. Cell culture (and adding any reagents to perform a bioassay) will be the only step required from the user, as electroporation and analysis of results will be performed automatically by computer-controlled setups in a plug-and-play manner. Electroporation will be performed by plugging the device (130) in a control setup (610) that will automatically apply an optimal electrical pulse to the cells (20), either preset or evaluated through electrical measurements done on the microelectrode array (130). A high content imaging tool (600) will automatically perform the analysis of the results. In embodiments, the electrodes may be connected in an array with individually addressable subjects.

### Example 1: High-throughput screening of nucleic acids using gene electrotransfer on microelectrode arrays

The aim of this experiment is to develop and demonstrate a high-throughput method for screening nucleic acids based on gene electrotransfer using microelectrode arrays (MEAs). This approach aims to overcome limitations of traditional bulk electroporation methods by enabling spatially-resolved transfection of multiple distinct nucleic acids on a single chip.

A microelectrode array chip is fabricated with electrodes made of ruthenium, which forms a native oxide layer on its surface. The chip contains 10,000 individually addressable microelectrodes in a 1 cm² area. To immobilize nucleic acids on the electrodes, the following procedure is used: First, the chip surface is cleaned using UV-ozone treatment for 10 minutes. Then, a 0.1% solution of poly(ethyleneimine) (PEI) in water is dispensed onto the chip surface and incubated for 30 minutes to allow electrostatic binding of the positively-charged PEI to the negatively-charged electrode oxide surface. After rinsing with deionized water, plasmid DNA encoding different fluorescent proteins is spotted onto individual electrodes using a microarray spotter, with each electrode receiving approximately 100 picoliters of a 100 ng/µL DNA solution. The chip is then air-dried and stored at 4°C until use.

To perform the transfection screening, HEK293 cells are seeded onto the MEA chip at a density of 100,000 cells/cm² in standard cell culture medium. The cells are allowed to adhere to the chip surface for 24 hours in a cell culture incubator. The chip is then connected to a custom electroporation control system. Electrical pulses (5 ms duration, 2 V amplitude) are applied to all electrodes simultaneously to trigger DNA release and cell electroporation. The cells are then returned to the incubator and cultured for 48 hours to allow for protein expression.

Fluorescence imaging of the entire chip is performed using an automated high-content imaging system. The results show distinct patches of fluorescence corresponding to the locations where different fluorescent protein-encoding plasmids had been spotted, indicating successful spatially-resolved transfection. Quantitative image analysis reveals that approximately 80% of cells directly above electrodes express the delivered genes, while minimal off-target transfection is observed. Cell viability assays show over 95% cell survival post-electroporation.

To demonstrate the high-throughput screening capability, a library of 1,000 different promoter sequences driving expression of a reporter gene is tested on a single chip. The entire workflow from cell seeding to data acquisition is completed within 3 days. Analysis of the fluorescence intensity data allows rapid identification of promoter sequences yielding high expression levels in the HEK293 cells.

This experiment successfully demonstrates a novel method for high-throughput nucleic acid screening using spatially-resolved electroporation on microelectrode arrays. The approach enables efficient testing of thousands of distinct nucleic acid constructs on a single chip with high transfection efficiency and cell viability. This platform has potential applications in fields such as gene therapy vector optimization, drug target discovery, and synthetic biology.

### Example 2: Screening of CRISPR guide RNAs using microelectrode array electroporation

This experiment aims to develop a high-throughput method for screening CRISPR guide RNAs (gRNAs) using microelectrode array (MEA) electroporation. The goal is to rapidly identify effective gRNAs for gene editing applications.

A microelectrode array chip is fabricated with 5,000 individually addressable platinum electrodes in a 0.5 cm² area. The chip surface is cleaned using a wet chemical process with piranha solution followed by deionized water rinses. A 0.5% solution of poly-L-lysine is then applied to the chip surface for 1 hour to create a positively charged layer for nucleic acid immobilization.

A library of 5,000 unique gRNA sequences targeting different sites in the human genome is synthesized. Each gRNA is complexed with Cas9 protein to form ribonucleoprotein (RNP) complexes. The RNP complexes are then spotted onto individual electrodes using a microarray spotter, with each electrode receiving approximately 50 picoliters of a 100 nM RNP solution. The chip is air-dried and stored at -20°C until use.

Human induced pluripotent stem cells (iPSCs) expressing a GFP reporter are seeded onto the MEA chip at a density of 200,000 cells/cm² in stem cell maintenance medium. After 6 hours of cell attachment, the chip is connected to an electroporation control system. Electrical pulses (2 ms duration, 1.5 V amplitude) are applied to all electrodes simultaneously to trigger RNP release and cell electroporation. The cells are then cultured for 72 hours to allow for gene editing to occur.

High-content imaging of the entire chip is performed to quantify GFP expression levels in cells above each electrode. Loss of GFP signal indicates successful gene editing by the delivered gRNA/Cas9 complexes. Image analysis reveals varying degrees of GFP knockout efficiency across the different gRNA sequences tested.

To validate the screening results, the top 50 gRNA sequences showing the highest GFP knockout efficiency are selected for further testing. These sequences are individually synthesized and tested in bulk cell populations using standard transfection methods. The results show a strong correlation between the MEA screening data and bulk validation experiments, confirming the reliability of the high-throughput screening approach.

The entire workflow from cell seeding to data acquisition is completed within 4 days, allowing rapid identification of highly effective gRNAs from a large library. This method demonstrates significant time and cost savings compared to traditional gRNA screening approaches.

In addition to GFP knockout efficiency, the MEA platform allows simultaneous assessment of cell viability and morphology changes induced by different gRNAs, providing valuable information on potential off-target effects.

This experiment successfully demonstrates the application of MEA-based electroporation for high-throughput screening of CRISPR guide RNAs. The approach enables efficient testing of thousands of gRNA sequences in stem cells with high spatial resolution and minimal reagent consumption. This platform has potential to accelerate the development of gene editing therapies and genomic engineering tools.

### Example 3: Screening of Protein Libraries Using Photoporation on a Microwell Array

A microwell array containing 1,000,000 individual wells in a 10 cm² area is fabricated using photolithography techniques. Each well has a diameter of 50 µm and a depth of 30 µm. The bottom of each well is coated with a photosensitive material.

A diverse library of 1,000,000 unique protein variants is generated using combinatorial protein engineering techniques. Each protein variant is individually dispensed into a separate microwell using a high-precision acoustic liquid handler, with each well receiving approximately 500 picoliters of a 1 mg/mL protein solution.

Human primary T cells are seeded onto the microwell array at a density of 500,000 cells/cm² in serum-free medium. The cells are allowed to settle into the microwells for 2 hours. A pulsed near-infrared laser system is then used to scan across the array, activating the photosensitive material at the bottom of each well. This generates localized shock waves that temporarily permeabilize the cell membranes, allowing the proteins to enter the cells.

After photoporation, the cells are cultured for 24 hours to allow for protein activity to manifest. The array is then subjected to a multiplexed cytokine release assay using a microengraved antibody array technique. This allows for simultaneous detection of multiple cytokines secreted by the cells in each individual microwell.

The resulting data is analyzed using a machine learning algorithm to identify protein variants that induce specific cytokine release profiles of interest. The top 1,000 candidates are selected for further characterization and optimization.

### Example 4: Iterative Optimization of Cell-Penetrating Peptides Using a Microelectrode Array and Machine Learning

A microelectrode array chip is fabricated with 100,000 individually addressable gold electrodes in a 2 cm² area. Each electrode has a diameter of 20 µm and is functionalized with a self-assembled monolayer terminating in maleimide groups.

An initial library of 100,000 cell-penetrating peptide (CPP) variants is designed based on known CPP sequences and rational design principles. Each peptide is synthesized with a C-terminal cysteine residue and a fluorescent tag. The peptides are individually spotted onto the electrodes, where they covalently bind to the maleimide groups.

Human neural stem cells are seeded onto the array at a density of 300,000 cells/cm² in neural induction medium. After 12 hours of adhesion, the array is connected to an electroporator. A series of optimized electric pulses is applied to trigger both the release of the peptides from the surface and the temporary permeabilization of the cell membranes.

The cells are cultured for 24 hours post-electroporation. High-content imaging is then performed to quantify the intracellular fluorescence intensity, providing a measure of peptide uptake efficiency. Additionally, a multiplexed RNA-FISH assay is conducted to measure the expression levels of key neural differentiation markers.

The resulting dataset, including peptide sequences, uptake efficiency, and marker expression profiles, is fed into a generative adversarial network (GAN) machine learning model. The GAN is trained to predict CPP performance based on sequence features and to generate novel CPP sequences optimized for both cellular uptake and the promotion of neural differentiation.

The top 10,000 Al-generated CPP sequences are synthesized and used for a second round of screening on the microelectrode array. This iterative process of experimentation, data analysis, and Al-driven design is repeated for five generations, progressively refining the CPP library for optimal performance in neural stem cell applications.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A method for screening entities (10) with different characteristics for the effect of these entities (10) on biological cells (20) of a biological cellular material, comprising:
a. Laterally constraining said entities (10) having different characteristics in different locations (40) of said biological cellular material, thereby preventing mixing of said entities (10) having different characteristics,
b. Applying a transfection activation field to said different locations (40) in such a way as to allow said entities (10) to be delivered into said biological cells (20) of said biological cellular material, thereby allowing said entities (10) to have an effect on said biological cells (20), and
c. Detecting said effect.

2. The method according to claim 1, wherein the different locations for constraining entities may be arranged with a density high enough such that a single biological cell (20) is positioned to be in simultaneous contact with two or more of said different locations.

3. The method according to claim 1 or claim 2, wherein the method further comprises measuring the permeability of the cell membrane at said different locations, and wherein the applied transfection activation field in step b is adjusted based on the measured permeability of the cell membrane, thereby allowing for real-time optimization of transfection parameters.

4. The method according to any one of the preceding claims, further comprising the step of creating a dataset (360) comprising said characteristics of said entities (10) associated with the effects detected for said entities (10).

5. The method according to claim 4, further comprising a step of designing new entities (380) based on the analysis of the dataset (360).

6. The method according to claim 5, further comprising a step of repeating steps a to c while using the new entities (380) in step a.

7. A system (560) for screening entities (10) with different characteristics for their effect on biological cells (20) of a biological cellular material, comprising:
- a device (570) comprising:
∘ A substrate (100) for supporting the biological cellular material,
∘ Constraining means (580) for laterally constraining said entities (10) over different locations (40) of said substrate (100) so as to prevent the mixing of entities (10) differing in their characteristics,
∘ Means (590) for applying a transfection activation field to the different locations (40) in such a way as to allow said entities (10) to be delivered into said biological cells (20) of said biological cellular material, thereby allowing said entities (10) to have an effect on these biological cells (20),
∘ A detector (600) for detecting said effect, and
- A control unit (610) configured to pilot the device (570) so that it
- Applies a transfection activation field to said different locations (40) in such a way as to allow said entities (10) to be delivered into said biological cells (20) of said biological cellular material, thereby allowing said entities (10) to have an effect on said biological cells (20), and
- Detects said effect.

8. The system (560) according to claim 7, further comprising a delivery unit (620), and wherein the control unit (610) is further configured to pilot the delivery unit (620) so that it delivers said entities (10) over said substrate (100) so that entities (10) differing in their characteristics are constrained over different locations (40) of the substrate (100) by the constraining means (580).

9. The system (560) according to claim 7 or claim 8, wherein said substrate (100) comprises a multielectrode array (130), wherein said means (590) for applying a transfection activation field are means (590) for applying an electrical field, and wherein said means (590) for applying an electrical field and/or said detector (600) comprises electrodes (140) of the multielectrode array (130).

10. The system (560) according to any one of claims 7 to 9, wherein the means (590) for applying the transfection activation field is configured to individually apply the transfection activation field to each of said different locations (40) in a controlled manner, taking into account one or more cellular health factors so as to enable delivery of the entities (10) into said biological cells (20) while preventing cellular death directly caused by the transfection activation field.

11. The system (560) according to any one of claims 7 to 10, wherein the constraining means (580) comprise compartments (120) on the substrate (100).

12. The system (560) according to any one of claims 7 to 11, wherein the detector (600) comprises an imaging unit (600), such as a fluorescence imaging unit (600) and/or a lens-free imaging unit (600).

13. The system (560) according to any one of claims 7 to 12, wherein the control unit (610) is further configured for collecting and processing data from the detector (600) to create a dataset that maps the observed effects to the corresponding locations (40) on the substrate (100).

14. The system (560) according to claim 13, wherein the control unit (610) is further configured for correlating the effects observed with the specific entities (10) constrained at those locations (40), thereby providing correlation between the characteristics of the entities (10) and their effects on the biological cells (20).

15. The system (560) according to claim 14, further comprising means (630) for designing new entities based on the correlation between the characteristics of the entities (10) and their effects on the biological cells (20).
